(19) Europäisches Patentamt
European Patent Office
Office européen des brevets

(11) Publication number: 0 200 916
B1

(12) **EUROPEAN PATENT SPECIFICATION**

(45) Date of publication of patent specification: 23.01.91

(51) Int. Cl.$^5$: C 08 J 3/02, C 08 L 83/04 // A61K7/00

(21) Application number: 86104397.4

(22) Date of filing: 01.04.86

# (54) Emulsification process and emulsions therefrom.

(30) Priority: 02.04.85 US 718974

(43) Date of publication of application: 12.11.86 Bulletin 86/46

(45) Publication of the grant of the patent: 23.01.91 Bulletin 91/04

(84) Designated Contracting States: BE DE FR GB IT

(56) References cited:
GB-A-2 079 300
US-A-4 177 177

(73) Proprietor: DOW CORNING CORPORATION
P.O. Box 1767
Midland Michigan 48640 (US)

(72) Inventor: Narula, Dipak
4512 North Saginaw Road, Apt. 608B
Midland, MI (US)

(74) Representative: Spott, Gottfried, Dr. et al
Patentanwälte Spott und Puschmann
Sendlinger-Tor-Platz 11
D-8000 München 2 (DE)

EP 0 200 916 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates generally to oil-in-water emulsions and to a process for their preparation using only nonionic surfactants. In a particular aspect the present invention relates to a process for emulsifying a bi-modal silicone fluid having a viscosity of at least 50 Pascal-Seconds (50,000 centipoise) at 25°C comprising substantial amounts of a polydimethylsiloxane gum and a volatile polydimethylsiloxane fluid.

Aqueous emulsions of silicones, including high viscosity silicones, have been prepared by emulsion polymerization of a silicon-containing monomer (Hyde et al., U.S. Patent No. 2,891,920; Findlay et al., U.S. Patent No. 3,294,725; and Axon, U.S. Patent No. 3,360,491) and by direct emulsification of a preformed silicone (Green, U.S. Patent No. 2,702,276; Volkmann et al., U.S. Patent No. 2,755,194 and Schneider et al., U.S. Patent No. 4,194,988). However, these processes use one or more surfactants of the ionic type for the formation and/or stabilization of the emulsion and are, therefore, not suitable for use in the many applications which require the absence of anionic and cationic species in the emulsion.

Evans et al., U.S. Patent No. 3,795,538 teach a process for emulsifying a polydiorganosiloxane fluid in water using only nonionic surfactants; however, bi-modal silicone emulsions are not contemplated therein.

Vanderhoff et al., U.S. Patent No. 4,177,177, teach a two-step process for emulsifying a polymer phase having a viscosity of less than about 10 Pascal-seconds (10,000 centipoise) in an aqueous medium containing at least one oil-in-water functioning emulsifier, in the presence of an additive to increase the stability of the final emulsion. However, patentees' process is not suitable for emulsifying a polymer phase having a viscosity of over 10,000 centipoise.

As disclosed in copending EP—A—193 485 (U.S. Application Serial No. 718,984) entitled "Substantive Skin Care Compositions Comprising a Polydimethylsiloxane", filed April 2, 1985), the incorporation of a polydimethylsiloxane having a viscosity of at least 30 Pascal-seconds (30,000 centipoise) into a skin care composition increases the skin-substantivity of a skin care component formulated therein. While such high viscosity silicones can be incorporated into a nonaqueous skin care composition with the aid of a solvent, therefore, their incorporation into an aqueous skin care composition has been prevented by the lack of a suitable aqueous emulsion thereof.

Copending EP—A—200 009 (U.S. Application Serial No. 718,985 entitled "Bi-modal Silicone Emulsions, Silicone Emulsification Process and Emulsions Therefrom", filed April 2, 1985), discloses aqueous emulsions of a polydimethylsiloxane fluid having a viscosity of up to 50 Pascal-seconds at 25°C and comprising a high viscosity silicone. These emulsions are useful as a substantivity aid in a skin care composition as noted above.

However, there is a need for aqueous emulsions comprising high viscosity silicones wherein the viscosity of the silicone exceeds 50 Pascal-seconds. In particular, there is a need for aqueous emulsions of a bi-modal silicone component having a viscosity as high as 2,000 Pascal-seconds and comprising as much as 50 percent silicone gum and 50 percent volatile silicone. The present invention provides these emulsions.

It is a particular object of the present invention to provide an emulsion of a bi-modal silicone fluid having a viscosity in excess of 50 Pascal-seconds (50,000 centipoise) and comprising substantial amounts of a volatile polydimethylsiloxane and a high viscosity, nonvolatile polydimethylsiloxane gum. It is further an object of this invention to provide a stable oil-in-water emulsion comprising a high viscosity silicone, and a process therefor, that comprises only nonionic surfactants. It is also an object of this invention to provide a process for preparing an aqueous emulsion of a high viscosity oil phase which does not require the use of additional heat to lower the viscosity of the oil phase, so that a stable balance of emulsion particle size and emulsion viscosity is obtained.

The present invention relates to a process for preparing an oil-in-water emulsion, and to the emulsions obtained thereby, said process comprising mixing an aqueous phase consisting of at least 10 parts by weight of water and an oil phase consisting of 100 parts by weight of a water-insoluble oil until the oil-in-water emulsion has been formed, said aqueous phase and/or said oil phase further containing, in total, at least 3 millimols of a nonionic primary surfactant having an HLB number of from 13 to 15; from 1 to 5 millimols, for every millimol of said nonionic primary surfactant, of a nonionic secondary surfactant having an HLB number of from 7 to 9; and at least 0.05 millimol of a nonionic tertiary surfactant having an HLB number of at least 16.

In a preferred embodiment, the oil phase has a viscosity of greater than 50 Pascal-seconds (50,000 centipoise) and the aqueous phase, comprising only a small amount of water, is admixed into the oil phase.

The process of this invention can be used to emulsify any water-insoluble oil into water. By the term oil, it is meant herein a liquid or semi-solid, polymeric material such as a freely flowing oligomer, a slowly flowing gum or a nonflowing gel, ranging in viscosity from a few millipascal-seconds to a few kilopascal-seconds and having a natural or synthetic origin. It is only necessary that the oil have a viscosity that permits its mixing with other emulsion components, such as surfactants and water, using typical emulsifying means.

The water-insoluble oil is preferably a hydrocarbon oil or a hydrocarbon-substituted siloxane oil or mixtures thereof; examples of which include, but are not limited to, mineral oil, petrolatum, polydimethylsiloxanes and their mixtures.

Herein the term silicone and the term polydimethylsiloxane are regarded as being synonymous and are used interchangeably. Me denotes the methyl radical.

A polydimethylsiloxane has the unit formula $Me_2SiO$- and consists of two or more of said units arranged in a cyclic and/or substantially linear molecular structure. Cyclic polydimethylsiloxanes have the formula $(Me_2SiO)_x$ wherein $x$ has a value of at least 3. Substantially linear polydimethylsiloxanes have the formula $R(Me_2SiO)_ySiMe_2R$ wherein $y$ has a value of at least 1 and R denotes a terminal radical such as hydroxy or alkoxy or hydrocarbyl, preferably having 1 to 6 carbon atoms, such as methyl, ethyl, vinyl and phenyl. Preferably R is methyl or hydroxy.

The silicone can consist of only a non-volatile silicone. Alternatively, the silicone can comprise a nonvolatile silicone and the normal amount, typically from 9 to 13 percent by weight, of volatile silicones that are produced during the normal siloxane equilibration process for preparing silicones. In addition, the silicone can consist of a nonvolatile silicone and a larger-than-normal amount of volatile silicone, such as 15, 20, 25 and more percent by weight.

Herein the distinction between a volatile silicone and a nonvolatile silicone is based on the normal boilding point of the silicone. Silicones which have a normal boiling point of less than 250°C are designated as volatile silicones. All other silicones are designated as nonvolatile silicones.

Examples of volatile silicones suitable for use in this invention include cyclopolydimethylsiloxanes having the formula $(Me_2SiO)_x$ wherein $x$ denotes 3, 4, 5 and 6 and methyl-terminated linear polydimethylsiloxanes having the formula $Me(Me_2SiO)_ySiMe_3$ wherein $y$ has a value of 1, 2, 3 and 4.

Examples of nonvolatile silicones for the purposes of this invention, include those having a viscosity at 25°C of at least 30 Pascal-seconds (30,000 centipoise), such as 30, 60, 100, 1000 Pascal-seconds (30,000, 60,000, 100,000, 1,000,000 centipoise) and more. For nonvolatile silicones having a viscosity exceeding 10 kilopascal-seconds (10 million centipoise), it is preferred to use the well-known units of plasticity number as delineated in ASTM D926—67. Thus, for silicone viscosities ranging from 10 to 20 to 40 to 80 kilopascal-seconds (10 million to 20 million to 40 million to 80 million centipoise) corresponding values of plasticity number for a substantially linear silicone will range from 130 to 146 to 165 to about 203, respectively. Correspondingly, the number average molecular weight will range from about 55,000 to about 350,000 as the viscosity ranges from 30 Pascal-seconds to 100 kilopascal-seconds. Of course, suitable nonvolatile silicones can contain trace amounts of polymer chain branching which will alter this viscosity-molecular weight relationship.

A preferred silicone for the process of this invention and for the composition of this invention is a bi-modal silicone. By a bi-modal silicone, it is meant herein a silicone that consists of a substantial amount, such as for example, from 25 to 99 percent by weight of a volatile silicone portion and from 1 to 74 percent by weight of a nonvolatile silicone portion having a viscosity of at least 30 Pascal-seconds at 25°C.

A bi-modal silicone is preferred for at least two reasons. First, the presence of a substantial amount of volatile, and hence low viscosity, silicone portion reduces the viscosity of the nonvolatile silicone, thereby allowing the use of a nonvolatile silicone portion having a viscosity of 10 kilopascal-seconds and more. Second, a bi-modal silicone component hhas a bi-modal efficacy in personal care compositions, i.e., the well-known efficacy of volatile silicones plus the above-noted substantivity-enhancing efficacy of a high viscosity silicone for certain skin care components, as well as other desirable effects such as water-repellency and lubrication.

A highly preferred silicone for the process and compositions of this invention is a bi-modal polydimethylsiloxane fluid having a viscosity of at least 30 Pascal-seconds at 25°C consisting of 50 to 90 parts by weight of a volatile polydimethylsiloxane portion F (octamethylcyclotetrasiloxane, decamethylcyclopentasiloxane, and their mixtures), and 10 to 50 parts by weight of a nonvolatile polydimethylsiloxane portion ϕ having an F having a normal boiling point of less than 250°C selected from cyclopolydimethylsiloxanes having 4 and 5 silicon atoms per molecule.

ϕ having a normal boiling point of 250°C or more and viscosity of at least 10 kilopascal-seconds at 25°C.

A bi-modal silicone comprising as little as 10 percent by weight of a silicon gum is a highly effective component for skin care formulations with respect to its substantivitiy-enhancing effect and its esthetic effects. However, bi-modal silicones having higher silicone gum contents, such as for example 25 to 50 percent by weight of silicone gum, are highly effective as components for skin care formulations from a cost and processing consideration as well. These bi-modal silicones having a higher gum content typically have a viscosity exceeding 50 Pascal-seconds (50,000 centipoise) at 25°C.

While the process of this invention is useful for emulsifying a bi-modal silicone having a viscosity of less than 50 Pascal-seconds (50,000 centipoise), and providing a stable emulsion thereof, the present process has particular utility for emulsifying a bi-modal silicone having a viscosity of greater than 50 Pascal-seconds (50,000 centipoise).

The process and compositions of this invention incorporate nonionic primary, secondary and tertiary surfactants to aid in the forming, stabilizing and thickening of the oil-in-water emulsions of this invention.

Nonionic surfactants are well known and need no detailed explanation herein. Nonionic surfactants suitable for use herein are principally of the ethoxylated substrate type therein the substrate is selected from hydrophobic alcohols, acids,

amides, esters and polyoxypropylenes. Suitable surfactants have ES, ESE and SES molecular structures wherein E denotes a polyoxyethylene moiety and S denotes a hydrophobic substrate. The primary, secondary and tertiary surfactants that are used in this invention can be of the same or different type, provided they are nonionic.

The primary surfactant that is used in this invention can be any nonionic surfactant having an HLB number of from 13 to 15; however, it is preferably an ethoxylated alkylphenol such as, for example, octylphenoxypolyethylene oxide containing an average of about 13 ethylene oxide units per molecule and having the CTFA name of octoxynol-13.

The reader is referred to "CFTA Cosmetic Ingredient Dictionary", Third Ed., 1982; the Cosmetic, Toiletry and Fragrange Association, Inc.; Washington, D.C. 20005, to further delineate the octoxynol nomenclature.

The HLB number of a surfactant is a well-known quantity and needs no explanation herein. The reader is referred to "McCutcheon's Detergents and Emulsifier"; Ridgewood, NJ; Allured Publishing Corp., for a comprehensive tabulation of surfactants in terms of HLB number, molecular structure, generic name and trade name.

The secondary surfactant that is used in this invention can be any nonionic surfactant having an HLB number of from 7 to 9; however, it is preferably an ethoxylated alkylphenol such as, for example, octylphenoxypolyethylene oxide containing an average of about 3 ethylene oxide units per molecular and having the CFTA name of octoxynol-3.

The tertiary surfactant that is used in this invention can be any nonionic surfactant having an HLB number of at least 16; however, it is preferably an ethoxylated saccharide such as, for example, polyethoxylated methyl glucose dioleate containing about 120 ethylene oxide units per molecular and having the CFTA name of PEF-120 methyl glucose dioleate.

The amounts of the primary and secondary surfactants that are used in this invention are related to each other and to the amount of water-insoluble oil that is used.

Thus, for every 100 parts by weight of water-insoluble oil at least 3 millimols of primary surfactant is used. Preferably, the amount of primary surfactant that is used in this invention will be from 5 to 50 millimols and most preferably from 10 to 25 millimols per 100 parts by weight of water-insoluble oil.

Herein the term millimols is to be taken in the same sense as the term parts by weight is taken. Thus, if the term parts by weight is applied on a gram basis, for example, the term millimol is to be applied on a milligram-mol basis.

The amount of secondary surfactant to be used in this invention is from 1 to 5, preferably 2 to 3, millimols for every millimol of primary surfactant that is used.

In terms of a preferred primary surfactant, i.e.,

$$C_8H_{17}C_6H_4(OCH_2CH_2)_nOH$$

wherein *n* has an average value of 13, and a preferred secondary surfactant, i.e.,

$$C_8H_{17}C_6H_4(OCH_2CH_2)_nOH$$

wherein *n* has an average value of 3, the amounts of each to use in this invention are at least 2.3, preferably 3 to 30 and most preferably 7.8 to 19.5, parts by weight of the former and at least 2.2, preferably 3.5 to 35 and most preferably from 11 to 19, parts by weight of the latter.

The amount of the tertiary surfactant that is to be used in this invention is surprisingly small, ranging up from about 0.05 millimol per 100 parts by weight of water-insoluble oil. While the actual upper and lower limits of the amount of tertiary surfactant have not been fully delineated, the practical limits thereof appear to be from about 0.05 to about 0.25 millimol per 100 parts by weight of water-insoluble oil.

In terms of a preferred tertiary surfactant, i.e., PEG-120 methyl glucose dioleate, the amount to be used in this invention ranges from about 0.3 part by weight, preferably from 0.3 to 1.5 parts by weight for every 100 parts by weight of the water-insoluble oil.

In the process and compositions of this invention, the amount of water to be used is not narrowly limited provided an emulsion can be formed with the amount used. For example, from 10 to 2,000 parts by weight of water for every 100 parts by weight of water-insoluble oil can be used. However, emulsions which are rich in water require longer periods of mixing than emulsions which are rich in water-insoluble oil. Consequently the amount of water to be used in this invention to form the emulsion is preferably limited to less than 350 parts by weight, and most preferably less than 100 parts by weight, per 100 parts by weight of water-insoluble oil. The water that is used in this invention should be clear and clean and has been preferably deionized or distilled.

In those instances wherein the oil phase has a viscosity of greater than 50 Pascal-seconds (50,000 centipoise) at 25°C, such as, for example, an oil phase consisting of a bi-modal silicone containing from 25 to 50 percent by weight of a silicone gum, it is preferred to limit the amount of water to from about 15 to 45 parts by weight, based on 100 parts by weight of water-insoluble oil.

The compositions of this invention can be diluted with water, if desired. For example, an emulsion composition of this invention containing 15 parts by weight water can be prepared and then diluted with water to an emulsion composition of this invention containing as much as 95 percent water.

The process and composition of this invention can further incorporate non-essential components such as thickeners, biostats, freeze-thaw stabilizers, colorants and odorants which are

commonly used in silicone-in-water emulsions and, particularly, in emulsions for personal care compositions.

The compositions of this invention are prepared by the process of this invention which generally comprises mixing suitable amounts of the above-described water-insoluble oil, the primary, secondary and tertiary surfactants and the water and thereafter further processing the mixture to form an emulsion having an average oil particle size of less than about 2 micrometers.

It should be noted that the emulsions of this invention that are prepared from a bi-modal silicone will have emulsion particles of widely varying sizes.

The emulsions of this invention are preferably prepared by mixing an aqueous phase comprising the water and the nonionic tertiary surfactant with an oil phase comprising the water-insoluble oil and the nonionic primary and secondary surfactants.

While it is possible to mix the aqueous phase with the oil phase in any manner, it is highly preferred to slowly admix the aqueous phase into the oil phase so that the mixture progresses from an oil-out to a water-out mixture.

The mixing is conducted until the mixture becomes a water-out emulsion and the desired size of oil particle is obtained.

Any mixing means can be used in the process of this invention provided only that it is capable of intimately mixing the components of the emulsion to be prepared. Examples of suitable mixing means include, but are not limited to, impeller mixers, sigma blade dough mixers and platenary mixers.

The compositions prepared by the method of this invention are expected to have the same utility as other oil-in-water emulsions of the art such as for lubricating or coating. The bi-modal emulsion compositions of this invention have particular utility in the formulations of personal care composition such as skin care, antiperspirant, deodorant and hygiene compositions.

The following examples are disclosed to further teach how to practice the present invention. They are not to be used to limit the invention which is properly delineated by the appended claims.

Viscosities were measured at 25°C in units of centipoise and were converted to Pascal-seconds Pa·s) for this disclosure by multiplying by 0.001. All parts and percentages are by weight. Plasticity numbers were measured according to ASTM D926-67.

Centrifuge stability was determined by spinning the emulsion at 3,000 revolutions per minute for 30 minutes, using a typical laboratory centrifuge. The sample was then visually examined for the presence of separation, settling and oiling. Results are qualitatively stated as none, slight or considerable with respect to each observation.

Freeze-thaw stability was determined by freezing the sample at −15°C for at least 4 hours and then melting the sample. The observations noted above were made after each freeze-thaw cycle.

Example 1

An oil phase was prepared by mixing a bi-modal polydimethylsiloxane fluid, 100 parts, having a viscosity of about 4 Pa·s and consisting of about 87 percent of a mixture of volatile cyclopolydimethylsiloxanes and about 13 percent of a nonvolatile polydimethylsiloxane having a plasticity number of about 150, 5.03 parts of octylphenoxypolyethylene oxide having the CFTA name octoxynol-13 and 4.85 parts of octylphenoxypolyethylene oxide having the CTFA name octoxynol-3 until homogeneous.

An aqueous phase was prepared by mixing 68.3 parts of water, 1.4 parts of PEG-120 methyl glucose dioleate (hereinafter PEG-120 MGD) and 2.5 parts of propylene glycol.

The aqueous phase was added to the oil phase using an air-driven stirrer at high speed. The resulting oil-in-water emulsion had a viscosity of 14.6 Pa·s, a particle size of less than 1 micrometer, as seen in an optical microscope, centrifuge stability and only slight oil separation after 5 freeze-thaw cycles. The siloxane content of this emulsion was about 60% based on siloxane plus water.

As a comparison, when this preparation was repeated using Methocel® (The Dow Chemical Company; Midland, MI) as a thickening agent instead of PEG-120 MGD an emulsion was obtained which had a large particle size distribution, a viscosity of 7.7 Pa·s and no freeze-thaw stability.

Example 2

The preparation of Example 1 was repeated except the aqueous phase consisted of 16.7 parts of water, 0.3 part of PEG-120 MGD and 2.5 parts of propylene glycol and the mixing was done with a sigma blade dough mixer for 4 hours. The resulting oil-in-water emulsion was a thick gel wherein at least 80% of the particles had a size less than 1 micrometer.

The thick gel was then diluted with 57.6 parts of a 2% solution of PEG-120 MGD in water. The resulting oil-in-water emulsion had a viscosity of 3.9 Pa·s and only slight oil separation after centrifuging or after 5 freeze-thaw cycles. The siloxane content of this emulsion was about 60%, based on siloxane plus water.

Example 3

An oil phase was prepared by mixing a bi-modal polydimethylsiloxane fluid, 100 parts, having a viscosity of about 250 Pa·s and consisting of about 70 percent of a mixture of volatile cyclopolydimethylsiloxanes and about 30 percent of a nonvolatile polydimethylsiloxane having a plasticity number of about 150, 11.36 parts of octoxynol-13 and 11.2 parts of octoxynol-3 until homogeneous.

An aqueous phase was prepared by mixing 17.3 parts of water, 0.35 part of PEG-120 MGD and 2.5 parts of propylene glycol as a freeze-thaw stability additive.

The aqueous phase was admixed into the oil

phase using a sigma blade dough mixer. The resulting water-in-oil emulsion was then treated with another 15.4 parts of water and 0.3 part of PEG-120 MGD for about 2 hours. The resulting clear gel emulsion was now the desired oil-in-water type and 95% of the particles thereof had a size of 1 micrometer or less.

When this preparation was repeated except that the aqueous phase contained 68.4 parts of water and 1.4 parts of PEG-120 MGD, the resulting emulsion had particles which were largely (75%) in the 1 to 2 micrometer range in size, and some were as large as 6 micrometers.

When the above-described preparation was repeated except that the aqueous phase consisted only of 69.8 parts of water and 2.5 parts of propylene glycol, i.e., the PEG-120 MGD was omitted, only 30% of the particles has a size of 2 micrometers or less and 70% of the particles has a size of 3 to 10 micrometers.

This example illustrates the improvement in particle size that can be obtained by the method of this invention comprising the use of a tertiary surfactant and, preferably a limited amount of water.

When the clear gel emulsion noted above was diluted with 57.5 parts of a 2% aqueous solution of PEG-120 MGD, an oil-in-water emulsion was obtained which had a viscosity of 26 Pa·s, centrifuge stability and only slight oiling after 5 freeze-thaw cycles.

Example 4

A bi-modal polydimethoxysiloxane fluid, 100 parts, having a viscosity of about 2 kPa·s and consisting of an equal weight mixture of the volatile and nonvolatile dimethylsiloxanes disclosed in Example 3 was thoroughly mixed with 19.1 parts of octoxynol-13 and 18.4 parts of octoxynol-3, using a sigma blade dough mixer until homogeneous. An aqueous phase consisting 15.5 parts of water, 0.3 part of PEG-120 MGD and 2.5 parts of propylene glycol was slowly added to the homogeneous oil-containing phase with mixing for 100 minutes. The resulting thick, white gel was an oil-in-water emulsion having particles of 1 micrometer or less in size. This gel was diluted with 56.7 parts of distilled water, using the same mixer, to provide an oil-in-water emulsion having a siloxane content of 55%, based on siloxane plus water, and a viscosity of 240 Pa·s..

Example 5

The preparation described in Example 4 was repeated except the aqueous phase consisted of 32.6 parts of water, 0.66 part of PEG-120 MGD and 2.5 parts of propylene glycol and the emulsion was mixed for 180 minutes instead of 100 minutes. The resulting oil-in-water emulsion was then diluted with 89.1 parts of water, using the same mixer, to provide an oil-in-water emulsion having a viscosity of 66 Pa·s and a siloxane content of 45%, based on siloxane plus water. Further dilution with water to 35% siloxane content reduced the viscosity to 1 Pa·s and provided an oil-in-water emulsion having stability to centrifugation and to 5 freeze-thaw cycles.

Examples 6 to 9

Four oil phases were prepared by thoroughly mixing, for each, 50 parts of the bi-modal polydimethylsiloxane fluid described in Example 1, 19.2 parts of octoxynol-13, 18.6 parts of octoxynol-3 and 50 parts of one of the following oils: (i) an equal weight mixture of white petrol petrolatum and mineral oil, (ii) an equal weight mixture of mineral oil and glycerine, (iii) an equal weight mixture of glycerine and volatile cyclopolydimethylsiloxane and (iv) an equal weight mixture of mineral oil and volatile cyclopolydimethylsiloxanes.

Four aqueous phases were prepared by mixing, for each, 47.6 parts of water, 0.97 part of PEG-120 MGD and 2.6 parts of propylene glycol.

Using an air-driven stirrer at high speed an aqueous phase was mixed into each of the four oil phases to provide, in the cases of (i) and (ii), a thick fluid emulsion and, in the cases of (iii) and (iv), a clear gel emulsion.

Each of the four emulsions was further diluted with 49.3 parts of water to provide emulsion compositions of this invention.

Example 10

A bi-modal polydimethylsiloxane fluid, 100 parts, having a viscosity of 44 Pa·s and consisting of 20 parts of the nonvolatile dimethylsiloxane and 80 parts of the volatile dimethylsiloxane described in Example 3 was thoroughly mixed with 7.6 parts of octoxynol-13 and 7.4 parts of octoxynol-3 using an air-driven stirrer at high speed to provide an oil phase.

An aqueous phase was prepared by mixing 68.3 parts of water, 1.4 parts of PEG-120 MGD and 2.5 parts of propylene glycol.

The aqueous phase was admixed with the oil phase, using the air stirrer, for 20 minutes to provide an oil-in-water emulsion having a viscosity of 18.5 Pa·s, centrifuge stability, freeze-thaw stability and a dual distribution of particles, the large particles being in the 1 to 3 micrometer size range.

Example 11

This example illustrates the preparation of a petrolatum-in-water emulsion without the use of additional heat.

An oil phase was prepared by mixing 100 parts of white petrolatum with 19.2 parts of octoxynol-13 and 18.6 parts of octoxynol-3.

An aqueous phase was prepared by mixing 33.7 parts of a 2% aqueous solution of PEG-120 MGD and 2.6 parts of propylene glycol.

The aqueous phase was thoroughly mixed into the oil phase to provide a white gel-like emulsion of the oil-in-water type. This emulsion was further diluted with 116.6 parts of the 2% aqueous solution of PEG-120 MGD.

Example 12

An oil phase was prepared by mixing 50 parts of white petrolatum, 50 parts of the bi-modal polydimethylsiloxane fluid described in Example 1, 19.2 parts of octoxynol-13 and 18.6 parts of octoxynol-3. An aqueous phase was prepared by mixing 38.7 parts of a 2% aqueous solution of PEG-120 MGD and 2.6 parts of propylene glycol. The aqueous phase was slowly and thoroughly admixed into the oil phase to provide a thick white gel, which was then diluted with 101.7 parts of water to provide an oil-in-water emulsion of this invention.

**Claims**

1. A process for preparing an oil-in-water emulsion, said process comprising mixing an aqueous phase consisting of at least 10 parts by weight of water and an oil phase consisting of 100 parts by weight of a water-insoluble oil until the oil-in-water emulsion has been formed, said aqueous phase and/or said oil phase further containing, in total, at least 3 millimols of a nonionic primary surfactant having an HLB number of from 13 to 15 per 100 gram water-insoluble oil, from 1 to 5 millimols, per millimol of said nonionic primary surfactant, of a nonionic secondary surfactant having an HLB number of from 7 to 9 and at least 0.5 millimol of a nonionic tertiary surfactant having an HLB number of at least 16 per 100 gram of water-insoluble oil.

2. A process according to claim 1 wherein the water-insoluble oil comprising a polydimethylsiloxane fluid having a viscosity of at least 30 Pascal-seconds at 25°C.

3. A process according to claim 2 wherein the polydimethylsiloxane fluid consists of:

(i) 10 to 50 percent by weight, based on the weight of the polydimethylsiloxane fluid, of a nonvolatile polydimethylsiloxane portion having a normal boiling point of 250°C or more and having a viscosity at 25°C of at least 10 kilopascal-seconds, and

(ii) 50 to 90 percent by weight, on the same basis, of a volatile polydimethylsiloxane portion having a normal boiling point of less than 250°C selected from cyclopolydimethylsiloxanes having 4 and 5 silicon atoms per molecule.

4. A process according to claim 3 wherein the oil phase contains all of the nonionic primary surfactant and all of the nonionic secondary surfactant and the aqueous phase contains all of the nonionic tertiary surfactant.

5. An oil-in-water emulsion consisting of 100 parts by weight of a water-insoluble oil, at least 0.05 millimol of a nonionic tertiary surfactant having an HLB number of at least 16 per 100 gram of water-insoluble oil, at least 3 millimols of a nonionic primary surfactant having an HLB number of from 13 to 15 per 100 gram of water-insoluble oil, from 1 to 5 millimols, per millimol of said primary surfactant, of a nonionic secondary surfactant having an HLB number of from 7 to 9, and at least 10 parts by weight of water.

6. A composition according to claim 5 wherein the water-insoluble oil comprises a polydimethylsiloxane fluid having a viscosity of at least 30 Pascal-seconds at 25°C.

7. A composition according to claim 6 wherein the polydimethylsiloxane fluid consists of:

(i) 10 to 50 percent by weight, based on the weight of the polydimethylsiloxane fluid, of a nonvolatile polydimethylsiloxane portion having a normal boiling point of 250°C or more and having a viscosity at 25°C of at least 10 kilopascal-seconds, and

(ii) 50 to 90 percent by weight, on the same basis, of a volatile polydimethylsiloxane portion having a normal boiling point of less than 250°C selected from cyclopolydimethylsiloxanes having 4 and 5 silicon atoms per molecule.

**Patentansprüche**

1. Verfahren zur Herstellung einer Öl-in-Wasser-Emulsion, wobei dieses Verfahren umfaßt, daß man eine wäßrige Phase, die aus mindestens 10 Gewichtsteilen Wasser besteht, und eine Ölphase, die aus 100 Gewichsteilen eines wasserunlöslichen Öls besteht, mischt, bis sich die Öl-in-Wasser-Emulsion gebildet hat wobei die wäßrige Phase und/oder die Ölphase weiterhin insgesamt mindestens 3 mMol pro 100 g wasserunlösliches Öl eines nichtionischen primären oberflächenaktiven Mittels mit einer HLB-Zahl von 13 bis 15, 1 bis 5 mMol pro mMol des nichtionischen primären oberflächenaktiven Mittels eines nichtionischen sekundären oberflächenaktiven Mittels mit einer HLB-Zahl von 7 bis 9 und mindestens 0,05 mMol pro 100 g wasserunlöslichem Öl eines nichtionischen tertiären oberflächenaktiven Mittels mit einer HLB-Zahl von mindestens 16 enthält, umfaßt.

2. Verfahren nach Anspruch 1, worin das wasserunlösliche Öl eine Polydimethylsiloxanflüssigkeit mit einer Viskosität von mindestens 30 Pascal-Sekunden bei 25°C umfaßt.

3. Verfahren nach Anspruch 2, woin die Polydimethylsiloxanflüssigkeit aus

(i) 10 bis 50 Gewichtsprozent, bezogen auf das Gewicht der Polydimethylsiloxanflüssigkeit eines nichtflüchtigen Polydimethylsiloxananteils mit einem normalen Siedepunkt von 250°C oder mehre und einer Viskosität von mindestens 10 Kilopascal-Sekunden bei 25°C und

(ii) 50 bis 90 Gewichtsprozent, bezogen auf dieselbe Basis, eines flüchtigen Polydimethylsiloxananteils mit einem normalen Siedepunkt von weniger als 250°C, ausgewählt aus Cyclopolydimethylsiloxanen mit 4 und 5 Siliciumatomen pro Molekül, besteht.

4. Verfahren nach Anspruch 3, worin die Ölphase das gesamte nichtionische primäre oberflächenaktive Mittel und das gesamte nichtionische sekundäre oberflächenaktive Mittel enthält und die wäßrige Phase das gesamte nichtionische tertiäre oberflächenaktive Mittel enthält.

5. Öl-in-Wasser-Emulsion, bestehend aus 100 Gewichtsteilen eines wasserunlöslichen Öls, mindestens 0,05 mMol pro 100 g des wasserunlöslichen Öls eines nichtionischen tertiären oberflächenaktiven Mittels mit einer HLB-Zahl von mindestens 16, mindestens 3 mMol pro 100 g an wasserunlöslichem Öl eines nichtionischen primären oberflächenaktiven Mittels mit einer HLB-Zahl von 13 bis 15, 1 bis 5 mMol pro mMol des primären oberflächenaktiven Mittels eines nichtionischen sekundären oberflächenaktiven Mittels mit einer HLB-Zahl von 7 bis 9 und mindestens 10 Gewichtsteilen Wasser.

6. Zusammensetzung nach Anspruch 5, worin das wasserunlösliche Öl eine Polydimethylsiloxanflüssigkeit mit einer Viskosität von mindestens 30 Pascal-Sekunden bei 25°C umfaßt.

7. Zusammensetzung nach Anspruch 6, worin die Polydimethylsiloxanflüssigkeit aus

(i) 10 bis 50 Gewichtsprozent, bezogen auf das Gewicht der Polydimethylsiloxanflüssigkeit, eines nichtflüchtigen Polydimethylsiloxananteils mit einem normalen Siedepunkt von 2502C oder mehr und mit einer Viskosität von mindestens 10 Kilopascal-Sekunden bei 25°C und

(ii) 50 bis 90 Gewichtsprozent, bezogen auf dieselbe Basis, eines flüchtigen Polydimethylsiloxananteils mit einem normalen Siedepunkt von weniger als 250°C, ausgewählt aus Cyclopolydimethylsiloxanen mit 4 und 5 Siliciumatomen pro Molekül, besteht.

**Revendications**

1. Un procédé pour préparer une émulsion d'huile dans l'eau, ledit procédé consistant à mélanger une phase aqueuse constituée d'au moins 10 parties en poids d'eau et une phase huileuse constituée de 100 parties en poids d'une huile insoluble dans l'eau, jusqu'à ce que l'émulsion d'huile dans l'eau ait été formée, ladite phase aqueuse et/ou ladite phase huileuse contenant de plus, au total, au moins 3 millimoles d'un agent tensio-actif non ionique primaire ayant un RHL de 13 à 15 pour 100 grammes d'huile insoluble dans l'eau, 1 à 5 millimoles, par millimole dudit agent tensio-actif non ionique primaire, d'un agent tensio-actif non ionique secondaire ayant un RHL de 7 à 9, et au moins 0,05 millimole d'un agent tensio-actif non ionique tertiaire ayant un RHL d'au moins 16 pour 100 grammes d'huile insoluble dans l'eau.

2. Un procédé selon la revendication 1, dans lequel l'huile insoluble dans l'eau comprend un fluide de polydiméthylsiloxane ayant une viscosité d'au moins 30 pascals-secondes à 25°C.

3. Un procédé selon la revendication 2, dans lequel le fluide de polydiméthylsiloxane est constitué de

(i) 10 à 50 pour cent en poids, sur la base du poids du fluide de polydiméthylsiloxane, d'une portion de polydiméthylsiloxane non volatil ayant un point d'ébullition normal de 250°C ou plus et ayant une viscosité à 25°C d'au moins 10 kilopascals-secondes, et

(ii) 50 à 90 pour cent en poids, sur la même base, d'une portion de polydiméthylsiloxane volatil ayant un point d'ébullition normal inférieur à 250°C, choisie parmi les cyclopolydiméthylsiloxanes ayant 4 et 5 atomes de silicium par molécule.

4. Un procédé selon la revendication 3, dans lequel la phase huileuse contient la totalité de l'agent tensio-actif non ionique primaire et la totalité de l'agent tensio-actif non ionique secondaire et la phase aqueuse contient la totalité de l'agent tensio-actif non ionique tertiaire.

5. Une émulsion d'huile dans l'eau constituée de 100 parties en poids d'une huile insoluble dans l'eau, au moins 0,05 millimole d'un agent tensio-actif non ionique tertiaire ayant un RHL d'au moins 16 pour 100 grammes d'huile insoluble dans l'eau, au moins 3 millimoles d'un agent tensio-actif non ionique primaire ayant un RHL de 13 à 15 pour 100 grammes d'huile insoluble dans l'eau, 1 à 5 millimoles, par millimole dudit agent tensio-actif primaire, d'un agent tensio-actif non ionique secondaire ayant un RHL de 7 à 9, et au moins 10 parties en poids d'eau.

6. Une composition selon la revendication 5, dans laquelle l'huile insoluble dans l'eau comprend un fluide de polydiméthylsiloxane ayant une viscosité d'au moins 30 pascals-secondes à 25°C.

7. Une composition selon la revendication 6, dans laquelle le fluide de polydiméthylsiloxane est constitué de

(i) 10 à 50 pour cent en poids, sur la base du poids du fluide de polydiméthylsiloxane, d'une portion de polydiméthylsiloxane non volatil ayant un point d'ébullition normal de 250°C ou plus et ayant une viscosité à 25°C d'au moins 10 kilopascals-secondes, et

(ii) 50 à 90 pour cent en poids, sur la même base, d'une portion de polydiméthylsiloxane volatil ayant un point d'ébullition normal inférieur à 250°C, choisie parmi les cyclopolydiméthylsiloxanes ayant 4 et 5 atomes de silicium par molécule.